(19) Europäisches Patentamt / European Patent Office / Office européen des brevets

(11) **EP 2 820 396 B1**

(12) **FASCICULE DE BREVET EUROPEEN**

(45) Date de publication et mention
de la délivrance du brevet:
**19.09.2018 Bulletin 2018/38**

(21) Numéro de dépôt: **13712876.5**

(22) Date de dépôt: **01.03.2013**

(51) Int Cl.:
**G01N 21/84** *(2006.01)*        **G01N 21/25** *(2006.01)*
**C08G 63/78** *(2006.01)*        **C08G 63/16** *(2006.01)*
**C07C 51/50** *(2006.01)*        **G01N 21/29** *(2006.01)*
**G01N 21/94** *(2006.01)*

(86) Numéro de dépôt international:
**PCT/FR2013/050438**

(87) Numéro de publication internationale:
**WO 2013/144471 (03.10.2013 Gazette 2013/40)**

(54) **METHODE DE MESURE DE LA STABILITE THERMIQUE D'UN ACIDE SUCCINIQUE CRISTALLIN DESTINE A LA FABRICATION DE POLYMERES**

VERFAHREN ZUR MESSUNG DER WÄRMESTABILITÄT EINES BERNSTEINSÄUREKRISTALLS ZUR HERSTELLUNG VON POLYMEREN

METHOD FOR MEASURING THE THERMAL STABILITY OF A SUCCINIC ACID CRYSTAL INTENDED FOR THE PRODUCTION OF POLYMERS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **02.03.2012 FR 1251924**

(43) Date de publication de la demande:
**07.01.2015 Bulletin 2015/02**

(73) Titulaire: **Roquette Frères**
**62136 Lestrem (FR)**

(72) Inventeurs:
• **FIEY, Guillaume**
**59000 Lille (FR)**
• **GUILLEMANT, Marilyne**
**62120 Aire sur la Lys (FR)**

• **ROTURIER, Jean-Michel**
**59280 Armentieres (FR)**
• **JACQUEL, Nicolas**
**59130 Lambersart (FR)**

(74) Mandataire: **Cabinet Plasseraud**
**31, rue des Poissonceaux**
**CS 40009**
**59044 Lille Cedex (FR)**

(56) Documents cités:
**EP-A1- 1 882 712    WO-A1-2011/064151**

• **TESTING METHODS FOR OPTICAL PROPERTIES OF PLASTICS: "JIS K 7105 / TESTING METHODS FOR OPTICAL PROPERTIES OF PLASTICS", JAPANESE INDUSTRIAL STANDARD, TOKYO, JP, vol. JIS K 7105, 1 janvier 1981 (1981-01-01), XP001247316,**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

La présente invention concerne une méthode colorimétrique permettant de qualifier l'acide succinique cristallin que l'on destine à la fabrication de polymères, notamment de type Poly Butylène Succinate (ou PBS).

Lorsqu'il est utilisé comme matériau de départ pour la fabrication de polymères, l'acide succinique doit être de haute pureté afin de maintenir le degré de polymérisation et surtout empêcher les phénomènes de coloration qui peuvent survenir aux hautes températures mises en oeuvre pour la réaction de polymérisation.

La présente invention présente ainsi un test colorimétrique permettant de déterminer la stabilité thermique de l'acide succinique utilisé pour la fabrication de polymères.

La présente invention permet à cette fin d'établir une corrélation entre la stabilité thermique de l'acide succinique cristallin et la couleur du polymère produit.

Cette méthode colorimétrique peut enfin être mise à profit pour identifier les impuretés impliquées dans les phénomènes de coloration.

L'acide succinique (ou acide butanedioïque) est un acide organique avec deux groupes carboxyles, de formule semi-développée COOH-CH$_2$-CH$_2$-COOH, qui intervient dans le métabolisme cellulaire comme intermédiaire métabolique du cycle de Krebs dans la mitochondrie.

Mais il trouve surtout de nombreuses applications dans les domaines cosmétiques, agroalimentaires, pharmaceutiques, textiles et dans les plastiques. Ainsi il est par exemple utilisé comme intermédiaire de synthèse des plastiques pour la fabrication de 1,4 butanediol, de tétrahydrofurane et de gammabutyrolactone.

Par ailleurs, les esters de l'acide succinique ont le potentiel d'être de nouveaux solvants « verts » qui peuvent substituer les solvants plus nuisibles pour l'homme et l'environnement.

Jusqu'à peu, l'acide succinique était essentiellement produit via des procédés basés sur la pétrochimie, mais considérés comme préjudiciables pour l'environnement. Des alternatives à ces procédés ont donc été développées.

La production d'acide succinique est ainsi actuellement réalisée à partir de matières premières renouvelables (en l'occurrence par le biais des processus de fermentation).

De ce fait, même si l'origine de l'acide succinique importe peu ici, la méthode de qualification de l'acide succinique cristallin selon l'invention a surtout été développée pour caractériser l'acide succinique biosourcé.

Diverses bactéries sont en effet connues pour leur capacité à produire de l'acide succinique par fermentation, tels qu'*Actinobacillus succinogenes, Mannheimia succiniciproducens* et *Escherichia coli,* aussi bien que des fungi tels qu'*Aspergillus niger* et *Saccharomyces cerevisiae.*

Divers procédés fermentaires sont également décrits pour assurer la production efficace en acide succinique et pour la récupération/purification de l'acide succinique du milieu de fermentation.

A ce propos, l'homme du métier recherche constamment de nouveaux procédés améliorés de purification d'acide succinique produit par fermentation.

En effet, les produits de fermentation contiennent généralement des quantités substantielles d'impuretés (débris de biomasse, sucres, acides aminés, oligoéléments, sels...) qui sont autant de précurseurs de coloration susceptibles par leur présence, même à l'état de traces dans le produit final, d'influer sur la qualité de l'acide succinique purifié, et par voie de conséquence, sur la qualité du polymère synthétisé à partir dudit acide succinique purifié.

Un certain nombre de procédés d'élimination desdits précurseurs de coloration ont donc été proposés :

- pour éliminer les impuretés azotées, il est proposé dans le brevet US 5.143.834 un procédé de purification qui consiste à soumettre notamment le milieu de fermentation contenant le succinate à une électrodialyse conventionnelle, celle-ci permettant également de concentrer le succinate en phase aqueuse.
- Pour éliminer les substances ionisées, il est décrit dans l'US 5.132.456 la mise en contact d'une matière première liquide contenant un sel d'acide succinique avec une résine échangeuse d'anions pour permettre à la résine d'adsorber l'acide succinique, et ainsi de le débarrasser de ses anions.

L'élution de l'acide succinique est réalisée ensuite par un solvant organique (ammoniaque ou analogues) ;

- Il est décrit dans le JP 62.238.232, une méthode comprenant une résine échangeuse de cations pour adsorber le contre-ion de l'acide succinique et recueillant l'acide succinique en tant que tel ;
- Quant à l'EP 1.669.459, il y est décrit l'utilisation d'une résine échangeuse de cations fortement acide de type H+ pour éliminer les impuretés de type ion sodium, ion potassium, ion magnésium et ion ammonium.

De même, cette résine permet l'élimination d'acides aminés tels la serine, l'acide glutamique, l'alanine, la valine, la méthionine et la tyrosine, qui sont difficiles à éliminer par simple cristallisation.

La concentration en cations autres que l'H+ dans l'effluent est alors ≤ 1.0%, de préférence ≤ 0.5% ;

- Dans le brevet EP 0 405.707, le niveau de pureté de l'acide succinique préparé pour des applications commerciales est indiqué par son contenu en impuretés azotées (< 1 %) et en sulfates ou autres ions contaminants (< 5 à 10 ppm).
- Dans le brevet EP 1.882.712, il est par ailleurs revendiqué :

  ◦ une teneur en atome d'azote de 0,01 ppm ou supérieure mais d'au plus 2 000 ppm par rapport à la quantité totale des matières premières,
  ◦ une teneur en atome de soufre de 0,01 ppm ou supérieure mais d'au plus 100 ppm par rapport à la quantité totale des matières premières.

Il apparait ainsi qu'il existe autant de seuils à respecter en certaines impuretés précurseurs de coloration qu'il existe de méthodes de purification conçues pour les éliminer.

Cependant, les méthodes classiques de quantification des impuretés précurseurs de coloration ne sont pas assez précises pour permettre la catégorisation des échantillons d'acide succinique en regard de leur qualité respective pour la fabrication de polymères.

De ce fait, à la connaissance de la société Demanderesse, aucune méthode colorimétrique fiable permettant de discriminer les lots d'acide succinique produit n'est décrite dans la littérature.

**De tout ce qui précède,** il résulte qu'il demeure un besoin non satisfait de disposer d'une méthode efficace, rapide et simple permettant de qualifier l'acide succinique cristallin destiné à la fabrication de polymères, notamment de type Poly Butylène Succinate (ou PBS).

La société Demanderesse a ainsi trouvé que ce besoin pouvait être satisfait par la mise au point d'une méthode colorimétrique rapide de détermination de la stabilité thermique de l'acide succinique produit, qui parvient d'ailleurs à s'affranchir de la connaissance de la nature des impuretés précurseurs impliquées.

Cette méthode consiste alors en la mesure de la coloration générée après traitement thermique de l'acide succinique cristallisé.

Cette mesure colorimétrique de l'acide succinique cristallin est réalisée sur un spectrocolorimètre (par exemple l'appareil Dataflash 100 commercialisé par la société DATACOLOR), en déterminant la moyenne de balance des blancs (L), des rouges (a) et des jaunes (b) d'un échantillon d'acide succinique cristallisé.

Comme il sera présenté ci-après, la stabilité thermique de l'acide succinique sera plus particulièrement exprimée par sa valeur de balance des jaunes (dit « *index b* »).

Le procédé de mesure de la stabilité thermique de l'acide succinique comprend alors les étapes selon la revendication 1.

La première étape du procédé conforme à l'invention consiste à préparer une poudre cristalline d'acide succinique présentant moins de 1 % de teneur en eau résiduelle.

La production de l'acide succinique proprement dite peut être réalisée par tout moyen connu par ailleurs de l'homme du métier : par voie chimique, mais surtout par voie fermentaire.

De manière avantageuse, l'acide succinique peut être préparé à partir de levures recombinantes, tel qu'enseigné dans les demandes de brevet WO 2009/011974, WO 2009/065780, WO 2009/065779, WO 2010/085731 ou WO 2010/118932.

L'acide succinique peut être extrait du milieu de fermentation par différentes méthodes, comme par exemple celle décrite par la société Demanderesse dans sa demande de brevet internationale WO 2011/064151.

Le procédé permet ainsi d'obtenir des cristaux d'acide succinique de haute pureté (> 99,5%/sec) - avec un rendement global de récupération > 90%.

La seconde étape du procédé conforme à l'invention consiste à placer un échantillon de ladite poudre cristalline dans une étuve à 220°C pendant 2 h ou 2h15.

Le procédé consiste plus particulièrement à préparer un échantillon d'exactement 10 g d'acide succinique cristallin, de le placer dans un tube résistant à haute température, et à le disposer à 220°C dans une étuve ventilée pendant 2 h ou 2h15.

La troisième étape du procédé conforme à l'invention consiste à broyer et tamiser la poudre cristalline ainsi traitée, de manière à ce que sa répartition granulométrique soit :

◦ comprise entre 0 et 10 %, de préférence comprise entre 4 et 6 % pour les particules de taille supérieure à 500 $\mu$m,
◦ comprise entre 20 et 40 %, de préférence comprise entre 25 et 35 % pour les particules de taille comprise entre 200 et 500 $\mu$m
◦ comprise entre 50 et 75 %, de préférence comprise entre 55 et 70 % pour les particules de taille inférieure à 200 $\mu$m.

Il est recommandé de laisser reposer le tube renfermant l'acide succinique ainsi thermiquement traité au minimum trois heures avant de procéder à la fabrication de la pastille qui sera lue sur le colorimètre.

La fabrication de ladite pastille ne peut être réalisée sur la poudre en tant que telle, étant donné les phénomènes de mottage subséquent au traitement thermique.

Il faut donc procéder à un broyage fin des agglomérats ainsi obtenus.

Le broyage est ainsi réalisé par tout moyen connu de l'homme du métier, i.e. de manière manuelle, dans un mortier, ou de manière mécanique, de manière à obtenir la répartition granulométrique de la poudre telle que présentée.

Cette répartition granulométrique est déterminée par tamisage en phase sèche sur tamiseur vibrant classiquement accessible à l'homme du métier, en suivant les prescriptions d'utilisation recommandée par le constructeur du tamis vibrant choisi.

Cette mise en forme facilite la fabrication de la pastille, selon les spécifications du constructeur du spectrocolorimètre.

La quatrième étape du procédé conforme à l'invention consiste à réaliser la mesure de la couleur dans un spectrocolorimètre et déterminer la valeur moyenne des jaunes (indice « b »).

La mesure colorimétrique est basée sur la théorie des couleurs opposées qui spécifie que les réponses des cônes (cellules de la rétine de l'oeil humain responsables de la vision des couleurs) aux couleurs rouge, verte et bleu sont recombinées en signaux opposés « noir-blanc », « rouge-vert » et « jaune-bleu » lorsque transmis au cerveau par le nerf optique.

Cette mesure repose sur les échelles de couleur largement utilisées dans les industries alimentaires et les industries des polymères, appelées échelles L, a, b de HUNTER.

Ce sont des échelles tridimensionnelles.

Les échelles de type L, a et b se définissent de la manière suivante :

- axe « L » (luminosité) : 0 correspond au noir, 100 correspond au blanc
- axe « a » (rouge-vert) : les valeurs positives dont attribuées au rouge ; les valeurs négatives sont attribuées au vert ; le 0 est la neutralité
- axe « b » (jaune-bleu) : les valeurs positives dont attribuées au jaune ; les valeurs négatives sont attribuées au bleu ; le 0 est la neutralité

L'indice « L » a donc une valeur comprise entre 0 et 100, tandis que les indices « b » et « a » n'ont pas de limitations numériques.

L'appareil de mesure est classiquement un spectrocolorimètre (permettant la mesure de réflexion à des longueurs d'onde comprises entre 400 et 700 nm), tel le Dataflash 100 commercialisé par la société DATACOLOR (Ouverture de mesure : 9 mm de diamètre » ; illuminant de lecture : « C2 Deg »).

Pour l'acide succinique cristallin, la mesure de l'indice « b » permet à lui seul de le qualifier pour son application polymère, comme il le sera démontré ci-après.

La méthode colorimétrique selon l'invention permet de catégoriser l'acide succinique cristallin produit.

Ainsi la présente invention a aussi pour objet un procédé de préparation d'un polymère de l'acide succinique, de préférence de poly butylène succinate (ou PBS), mettant en jeu, comme matière première, un acide succinique sélectionné à l'aide du procédé de mesure de stabilité thermique décrit ci-dessus. Ce procédé de préparation d'un polymère de l'acide succinique est notamment caractérisé en ce que l'acide succinique présente une valeur moyenne des jaunes inférieure ou égale à 2.

Pour le démontrer, la société Demanderesse a entrepris deux séries d'expérimentation ayant pour objectif de tester l'impact de la coloration du monomère sur le niveau de coloration de polymères produit à partir de différents lots d'acide succinique, d'origine chimique et surtout fermentaire, la qualité chimique étant considéré ici témoin positif :

- détermination de l'indice « b » pour différents lots d'acide succinique produits par voie fermentaire à partir de souches de microorganismes recombinants de type *E. coli* ou *S. cerevisiae,* et mesure de la coloration du polymère correspondant.

Cette première expérimentation a ainsi permis de définir une valeur seuil de l'indice « b » de 1,9, en deçà de laquelle l'acide succinique cristallin peut être retenu pour l'application polymère PBS en particulier.

- détermination qualitative et quantitative des impuretés susceptibles d'agir comme précurseurs de coloration de l'acide succinique produit par voie fermentaire.

Quant à la coloration du polymère produit à partir de l'acide succinique (en l'occurrence ici le PBS), elle est classiquement déterminée sur un spectrophotomètre (par exemple DYK Gardner TCS II - angle d'observation de 10°, illumination D65), en déterminant également la moyenne de balance des blancs (L), des rouges (a) et des jaunes (b).

La coloration du polymère sera exprimée alors par sa valeur de balance des jaunes (dite ici « *Yellow index* »).

**Exemple 1** : Production par voie fermentaire, extraction et purification de l'acide succinique

Une première série de fermentations par une *E. coli* recombinante (ici la souche SBS550MG - pHL413 décrite dans Sanchez et al., Metabolic Engineering, 7 (2005) 229-239, et dans les documents US 7.223.567 et US 2005/0042736) est réalisée.

Une seconde série de fermentations l'est également, par une souche recombinante de *S. cerevisiae* (ici celle décrite dans la demande de brevet WO 2009/065778).

L'extraction des succinates et la purification de l'acide succinique sont réalisées à partir de ces milieux de fermentations par la succession des étapes suivantes :

*- Elimination des impuretés organiques insolubles (biomasse et débris cellulaires)*

L'élimination est réalisée par filtration tangentielle à flux tangentiel sur membrane présentant un diamètre de pore de 100 nm, entre 40 et 80°C (membrane de type céramique de 3,5 mm de diamètre de canal).

La température est maintenue préférentiellement à 60°C avec une pression transmembranaire de 1 bar et une diafiltration avec 20% d'eau déminéralisée.

Dans ces conditions, le flux est d'environ 90 L/h/m$^2$ et le perméat obtenu est limpide et brillant. Le perméat contient encore plus de 6000 ppm d'impuretés organiques solubles, ici de l'azote organique soluble

*- Elimination des impuretés organiques solubles (protéines solubles résiduelles)*

Cette élimination consiste à adsorber l'azote organique sur du charbon actif ou à le dénaturer avant de l'éliminer par filtration. Dans le cas de la dénaturation, celle-ci peut être thermique ou osmotique.

On utilise une zone de contact à 80°C permettant de floculer les protéines pendant 10-15 min. La solution est ensuite filtrée à travers un filtre de diamètre de pore 0,22 $\mu$m.

Cette étape d'élimination d'azote organique devient optionnelle dans le cas où la microfiltration est réalisée à 80°C. En effet, il y a alors simultanément dénaturation de l'azote organique et rétention sur la membrane de microfiltration.

*- Chélation et Acidification*

La concentration maximale admissible en alimentation de l'électrodialyse bipolaire (EDB) en termes de cations divalents (Ca$^{2+}$, Mg$^{2+}$...) est de 5 ppm. En effet, les cations divalents présents vont réagir avec les ions hydroxyles au niveau des membranes d'électrolyse de l'eau pour former des sels très peu solubles, qui cristallisent dans les membranes et les rendent perméables.

Afin de protéger l'intégrité des membranes d'EDB, une étape de chélation est réalisée pour atteindre le seuil de sécurité de 5 ppm.

Cette étape consiste à complexer les cations divalents à l'aide de fonctions aminophosphoniques (Purolite S940, Amberlite IRC747...) ou diacétiques (Purolite S930, Lewatit TP208) greffées sur résine échangeuse de cations.

Pour ce faire la solution est alimentée à 60°C, au débit de 2 BV/h. Dans ces conditions, le volume de solution traitée peut atteindre 30 à 40 fois le volume du lit de résine.

Une fois débarrassée de ces cations divalents, la solution peut être acidifiée par EDB. Le module utilisé est un *stack* de la société EURODIA : EUR6.

Ce *stack* est constitué de deux types de membranes :

-   les membranes bipolaires qui permettent l'électrolyse de l'eau (H2O $\rightarrow$ H$^+$ + OH$^-$) et l'acidification des succinates,
-   les membranes cationiques, qui permettent le transfert sélectif des cations monovalents.

L'électrolyse et le transfert des cations sont assurés par à une différence de potentiel appliquée au système grâce à un générateur.

Le sel de succinate est alors acidifié au cours du temps par les ions H$^+$ libérés par l'électrolyse de l'eau. De la même manière, les cations monovalents sont alcalinisés par les ions hydroxyles après migration à travers les membranes cationiques pour redonner une base recyclable en fermentation.

Le flux de cations transférés est d'environ 22 eq/h/m$^2$ pour un taux de conversion de 90%, ce qui équivaut à un pH final d'environ 3,5.

La finition de l'acidification s'effectue sur résine cationique forte (PUROLITE C150). En effet, les derniers équivalents sont très difficiles à transférer et la consommation énergétique de l'opération est alors fortement accrue (limitation principalement due à la pression osmotique).

Le traitement s'effectue préférentiellement à 40°C et à 2 BV/h. Le volume de solution traitée est alors d'environ 10-15 BV.

Le pH de la solution après traitement sur résine cationique est égal à 2 et contient l'acide succinique sous sa forme peu soluble (acide libre).

### - *Cristallisation*

La solution acidifiée est concentrée par évaporation de l'eau sur un évaporateur à film tombant de type WIEGAND®. Le facteur de concentration est de l'ordre de 5 à 10 selon la concentration initiale en acide succinique.

Il est ici égal à 8 pour atteindre la sursaturation.

La concentration de la solution est alors de 420 g/L à 80°C, ce qui correspond à la sursaturation de la solution.

La solution est ensuite refroidie par contact direct de 80 à 20°C avec une vitesse de 5°C/h.

La cristallisation démarre spontanément dès le début du refroidissement mais un ensemencement peut être réalisé pour maitriser au mieux les propriétés physiques de l'acide succinique cristallisé.

Après séparation sur une centrifugeuse ROUSSELET® et un lavage avec un volume d'eau déminéralisée par volume de gâteau, les cristaux sont séchés.

A cette étape, le rendement de cristallisation est > 85% pour une pureté en acide succinique de 99,7%/sec.

### - *Remise en solution*

La remise en solution des cristaux s'effectue dans l'eau déminéralisée à 60°C plutôt qu'à 20°C, afin de réduire la consommation d'eau déminéralisée.

Dans la même optique, la liqueur mère et/ou les eaux de lavage des cristaux de haute pureté peuvent également être recyclées à cette étape. Le rendement global en acide succinique est alors également optimisé.

### - *Traitement de finition*

Cette étape est constituée d'un traitement de décoloration et d'une étape de déminéralisation. La décoloration peut s'effectuer par charbon actif ou ozonation.

Le traitement au charbon actif présente l'intérêt majeur de fixer l'acide orotique, impureté azotée très peu soluble et cristallisant avec l'acide succinique.

En batch, la dose de charbon actif NORIT SX$^+$ est de 1% pour rapport à l'acide succinique. Après 1h de réaction à 60°C, la solution est filtrée sur un filtre à bougies de 3$\mu$m.

La solution est ensuite traitée en déminéralisation à 2BV/h à 60°C.

Réalisé successivement sur résine cationique forte PUROLITE® C150 puis anionique faible DAION® WA30, le traitement permet d'éliminer les traces d'anions et cations minéraux d'une part et l'acide fumarique d'autre part. De plus, la résine anionique faible permet une décoloration supplémentaire.

Après traitement sur résine anionique faible, la teneur en acide fumarique peut être diminuée d'un facteur 20 et la coloration résiduelle est nulle.

### - *Cristallisation*

La solution purifiée peut alors être cristallisée dans les mêmes conditions qu'à l'étape précédente

Le rendement de cette étape de cristallisation est également > 85%. La liqueur mère et les eaux de lavage sont recyclées au niveau de la dissolution des cristaux de qualité technique. Le rendement global de cette étape peut ainsi tendre vers 100%. La pureté des cristaux est de plus de 99,8 %.

**Exemple 2** : **Mesure colorimétrique de l'acide succinique produit par fermentation de microorganismes recombinants**

3 fermentations successives ont été réalisées pour la souche de *E. coli* (Essais « A » à « C ») ; 6 pour la souche de *S. cerevisiae* (Essais « D » à « I ») et l'acide succinique extrait et purifié dans les conditions de l'exemple 1.

Les 9 échantillons de poudre cristalline d'acide succinique sont ensuite analysés en suivant la méthode conforme à l'invention.

10 g de chacun de ces échantillons ont été placés dans une étuve à 220°C pendant 2h 15 ; La poudre récupérée a été broyée et analysée en spectrocolorimètre conformément à la méthode de l'invention.

Le tableau 1 suivant présentent les valeurs des indices « L », « a » et « b » telles qu'obtenues.

Tableau 1

| Echantillons | « L » | « a » | « b » |
|---|---|---|---|
| Acide succinique « chimique » | 98.0 | 0.00 | 1.3 |
| « A » | 91.1 | 0.99 | 6.2 |
| « B » | 97.3 | 0.19 | 1.8 |
| « C » | 97.1 | 0.14 | 1.9 |
| « D » | 96.9 | 0.00 | 2.1 |
| « E » | 96.5 | 0.29 | 2.3 |
| « F » | 97.4 | 0.16 | 1.7 |
| « G » | 97.3 | 0.14 | 1.7 |
| « H » | 98.1 | 0.11 | 1. 1 |
| « I » | 97.3 | 0.23 | 1.9 |

Par rapport à l'échantillon d'acide succinique chimique de référence (commercialisé par la société SIGMA), il apparaît ici que seul l'échantillon « H » aurait la qualité colorimétrique requise.

Pour le démontrer, des essais de polymérisation avec ces différentes qualités ont été entrepris.

Des échantillons de PBS sont synthétisés via une réaction de polycondensation en deux étapes, dans un réacteur en acier inoxydable de 7,5 l équipé d'un système de chauffage, d'un agitateur mécanique, d'une colonne de distillation, d'une ligne de vide et d'une entrée d'azote gazeux.

On charge le réacteur avec 1889 g (16 moles d'acide succinique) et 1513,7 g (16,8 moles) de 1,4 butanediol (BDO).

Le mélange réactionnel est ensuite chauffé à 225°C sous 2 bars de pression d'azote et agité à une vitesse constante de 150 rpm.

Un mélange d'eau et de tétrahydrofurane (1 à 2,5 mol % de THF) produit de l'estérification et de la cyclisation du BDO sont éliminés du réacteur par la colonne à distiller.

Le taux d'estérification est estimé par la quantité du distillat collecté.

Dans une deuxième étape, la pression est réduite à 0,7 mbars en 120 minutes.

La réaction est catalysée par 3,914 g de Ti(OBu)$_4$ (Ti = 200ppm), qui sont ajoutés au réacteur durant l'étape de décompression (environ 20 mbars) pour éviter le plus possible le contact avec l'eau résiduelle.

Les conditions de pression sont maintenues durant 3,8 heures.

Le polymère est retiré du réacteur et immergé dans de l'eau. Environ 15 mg de granulés de PBS sont obtenus après granulation.

Le degré de coloration du polymère est ensuite déterminé sur des plaques polymérisées présentant la même épaisseur (de l'ordre de 3 mm) à l'aide d'un spectrophotomètre DYK GARDNER TCS II (angle d'observation de 10°, illuminant D65).

Les résultats sont également exprimés par les indices « L », « a » et « b », et plus particulièrement par la mesure de l'indice du jaune (encore appelé « yellow index » - abréviation : YI).

Le YI est en fait calculé à partir de l'équation suivante (ASTM D1925) :

$$YI = \frac{100 \times (1.28 X_{CIE} - 1.06 Z_{CIE})}{Y_{CIE}}$$

Le tableau 2 suivant renseigne les YI des polymères produits à partir de deux différents échantillons d'acide succinique biosourcé d'indice « b » connu, de part et d'autre du seuil de valeur de l'indice « b » établi ci-avant, i.e. indice « b » = 2.

Tableau 2

| PBS préparé à partir de : | Indice « b » | YI |
|---|---|---|
| | 1,3 | 7,8 |
| **2 lots d'acide succinique produit par voie chimique** | 1,3 | 6,1 |
| **« H »** | 1,1 | 6,5 |
| **« F »** | 1,7 | 9,8 |
| **« D »** | 2,1 | 11 |

Un PBS est considéré correct par l'homme du métier si son YI est < 10.

Ces résultats permettent de confirmer la bonne qualité de l'acide succinique « H », et de montrer que l'on peut aller jusqu'à la valeur d'indice « b » de 1,7 pour produire une qualité colorimétrique de PBS acceptable.

**Exemple 3 : Confirmation de la valeur seuil de l'indice « b » de l'acide succinique bio sourcé : enrichissement d'un acide succinique de référence avec diverses impuretés de nature et quantité définies**

Les impuretés susceptibles d'agir comme précurseurs de coloration classiquement identifiés dans les milieux de fermentation sont notamment des substances résiduelles de type hydrates de carbone, azote minéral ou organique, soufre minéral et quelques acides organiques coproduits par les souches.

Afin de tester la robustesse de la méthode de qualification de l'acide succinique cristallin de l'invention, et l'usage prédictif que l'on pourrait en faire, on enrichit artificiellement un acide succinique d'origine chimique (indice « b » de référence = 1,3) ou biosourcé (acide succinique correspondant au lot « H » de l'exemple 1 - indice « b » de référence = 1,1) avec des impuretés représentatives de ce que l'on trouve dans lesdits milieux de fermentation avant de réaliser le traitement en étuve à 200°C.

Les contaminants choisis sont les suivants (testés aux doses indiquées) :

- hydrate de carbone : glucose à des concentrations de l'ordre de 100 ppm ;
- azote organique : valine à une concentration de 200 ppm pour l'acide succinique d'origine chimique ; 1000 ppm pour celui biosourcé ;
- soufre : $Na_2SO_4$ à la concentration de 200 ppm pour l'acide succinique d'origine chimique ; 1000 ppm pour celui biosourcé ;
- acides organiques : acide fumarique, acide malique à la concentration de 1000 ppm, testés uniquement avec l'acide succinique d'origine chimique ;
- acide organique soufré : acide orotique à la concentration de 1000 ppm, testé uniquement avec l'acide succinique d'origine chimique ;
- Soufre et azote : sulfate d'ammonium à 1200 ppm, testé uniquement avec l'acide succinique biosourcé.

Les résultats obtenus sont présentés dans le tableau 3 suivant.

Tableau 3

| Echantillons | Index « b » | Δ (b - b.ref) |
|---|---|---|
| AS chimique + 100 ppm de glucose | 3,3 | 2 |
| Essais « H » + 100 ppm de glucose | 3,2 | 2,1 |
| AS chimique + 200 ppm de valine | 1,5 | 0,2 |
| Essais « H » + 1000 ppm de valine | 1,3 | 0,2 |
| AS chimique + 100 ppm de glucose + 200 ppm de valine | > 5 | > 4 |
| AS chimique + 200 ppm de $Na_2SO_4$ | 1,3 | 0 |
| Essais « H » + 1000 ppm $Na_2SO_4$ | 1,2 | 0,1 |
| AS chimique + 1000 ppm d'acide fumarique | 1,7 | 0,5 |
| AS chimique + 1000 ppm d'acide malique | 1,5 | 0,2 |

(suite)

| Echantillons | Index « b » | Δ (b - b.ref) |
|---|---|---|
| AS chimique + 1000 ppm d'acide orotique | 2 | 0,7 |
| Essais « H » + 1200 ppm de sulfate d'ammonium | 2 | 0,9 |

Ces résultats démontrent :

- L'importance du glucose résiduel seul dans la stabilité thermique de l'acide succinique biosourcé ;

- les contaminants azotés ne provoquent qu'une légère altération de la stabilité thermique de l'acide succinique ;

- la combinaison des contaminants azotées et soufrées amplifient le phénomène de coloration

La société Demanderesse va à ce propos à l'encontre d'un préjugé technique qui veut que seules les impuretés azotés et soufrés doivent être contrôlées (cf. le brevet EP 1.882.712 cité ci-avant) ;

- les dérivés de l'acide succinique (acide malique et fumarique) ainsi que le soufre ($Na_2SO_4$) n'ont pas d'impact significatif sur la coloration de l'acide succinique chimique pris comme référence,

- les contaminants azotés ne provoquent qu'une légère altération de la stabilité thermique de l'acide succinique.

Pour compléter ce travail, on a procédé à la fabrication du PBS à partir de différents lots d'acide succinique chimique enrichis en impuretés (ceux-ci-dessus et d'autres échantillons préparés spécialement pour cette deuxième série d'essais).

Le tableau 4 suivant présente les résultats de YI mesurés pour chacun polymères produits.

Tableau 4

| PBS produit avec | N (ppm) | S (ppm) | indice « b » du monomère | « Yellow index » du PBS |
|---|---|---|---|---|
| un premier acide succinique chimique de référence | 0 | 0 | 1,3 | 7,8 |
| un second acide succinique chimique de référence | 0 | 0 | 1,3 | 6,1 |
| + 1000 ppm d'acide fumarique | 0 | 0 | 1,7 | 9,8 |
| + 1000 ppm d'acide malique | 0 | 0 | 1,5 | 4,6 |
| + 1000 ppm d'acide orotique | 179 | 0 | 2 | 15,7 |
| + 200 ppm de $Na_2SO_4$ | 0 | 45 | 1,3 | 2,8 |
| + 200 ppm de valine | 24 | 0 | 1,5 | 3,9 |
| + 20 ppm de glucose | 0 | 0 | 2,5 | 11,3 |
| + 100 ppm de glucose | 0 | 0 | 3,3 | 18,1 |
| + 100 ppm de glucose + 200 ppm de valine | 24 | 0 | 4 | 15,5 |

Ces premiers résultats confirment l'influence des impuretés glucose et acide orotique sur la qualité du polymère, comme sur celle du monomère.

Par ailleurs, en établissant la courbe traçant les valeurs de l'indice « b » du monomère en fonction du YI du polymère, et prenant toujours pour référence un PBS de coloration correcte quand son YI est ≤ 10, on trouve une valeur seuil pour l'indice « b » voisine de 2.

**Revendications**

1. Procédé de mesure de la stabilité thermique de l'acide succinique **caractérisé en ce qu'**il comprend les étapes suivantes :

   **1)** préparer une poudre cristalline d'acide succinique présentant moins de 1 % de teneur en eau résiduelle,
   **2)** placer 10 g de ladite poudre cristalline dans une étuve à 220°C pendant 2 h ou 2h15,
   **3)** broyer et tamiser la poudre cristalline ainsi traitée, de manière à ce que sa répartition granulométrique soit :

   ◦ comprise entre 0 et 10 %, de préférence comprise entre 4 et 6 % pour les particules de taille supérieure à 500 $\mu$m,
   ◦ comprise entre 20 et 40 %, de préférence comprise entre 25 et 35 % pour les particules de taille comprise entre 200 et 500 $\mu$m
   ◦ comprise entre 50 et 75 %, de préférence comprise entre 55 et 70 % pour les particules de taille inférieure à 200 $\mu$m,

   4) réaliser la mesure de la couleur dans un spectrocolorimètre d'une pastille fabriquée sur la poudre broyée et tamisée et déterminer la valeur moyenne des jaunes (indice « b »).

2. Procédé de préparation d'un polymère de l'acide succinique, de préférence de poly butylène succinate (ou PBS), mettant en jeu, comme matière première, un acide succinique sélectionné à l'aide du procédé selon la revendication 1.

3. Procédé selon la revendication 2, **caractérisé en ce que** l'acide succinique présente une valeur moyenne des jaunes inférieure ou égale à 2.


**Patentansprüche**

1. Verfahren zur Messung der thermischen Stabilität von Bernsteinsäure, **dadurch gekennzeichnet, dass** es die folgenden Schritte umfasst:

   1) Zubereiten eines kristallinen Bernsteinsäurepulvers, welches weniger als 1% Restwassergehalt aufweist,
   2) Platzieren von 10 g des kristallinen Pulvers in einer Wärmekammer bei 220 °C über 2 h oder 2h15,
   3) Zerkleinern und Sieben des derart behandelten kristallinen Pulvers, sodass die Verteilung der Partikelgröße wie folgt ist:

   • 0 bis 10 %, bevorzugt 4 bis 6 % Partikel größer 500 $\mu$m,
   • 20 bis 40 %, bevorzugt 25 bis 35 % Partikel 200 bis 500 $\mu$m,
   • 50 bis 75 %, bevorzugt 55 bis 70 % Partikel kleiner 200 $\mu$m,

   4) in einem Spektralphotometer Durchführen der Messung der Farbe einer auf zerkleinertem und gesiebten Pulver hergestellten Pastille und Bestimmen des Mittelwertes der Gelbtöne (Index ‚b').

2. Verfahren der Zubereitung eines Polymers der Bernsteinsäure, bevorzugt Polybutylensuccinat (oder PBS), unter Einsatz einer Bernsteinsäure als Ausgangsstoff, die mittels eines Verfahrens nach Anspruch 1 gewählt wird.

3. Verfahren nach Anspruch 2, **dadurch gekennzeichnet, dass** die Bernsteinsäure einen Mittelwert an Gelbtönen kleiner gleich 2 aufweist.


**Claims**

1. Method for measuring the heat stability of succinic acid, the method comprising the following steps:

   1) preparing a crystalline powder of succinic acid having a residual water content of less than 1%,
   2) placing a sample of said crystalline powder in an oven at 220°C for 2 h or 2h15,
   3) milling and sieving the crystalline powder thus treated, in such a way that its particle size distribution is:

○ between 0 and 10%, preferably between 4 and 6%, for the particles with a size greater than 500 μm,

○ between 20 and 40%, preferably between 25 and 35%, for the particles with a size between 200 and 500 μm,

○ between 50 and 75%, preferably between 55 and 70%, for the particles with a size less than 200 μm,

4) measuring the colour of a pellet made of the milled and sieved powder in a spectrocolorimeter and determining the mean yellow value (index "b").

2. Method for preparing a succinic acid polymer, preferably poly(butylene succinate) (or PBS), using, as raw material, a succinic acid selected by means of the method according to Claim 1.

3. Method according to Claim 2, **characterized in that** the succinic acid has a mean yellow value of less than or equal to 2.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5143834 A
- US 5132456 A
- JP 62238232 B
- EP 1669459 A
- EP 0405707 A
- EP 1882712 A
- WO 2009011974 A
- WO 2009065780 A
- WO 2009065779 A
- WO 2010085731 A
- WO 2010118932 A
- WO 2011064151 A
- US 7223567 B
- US 20050042736 A
- WO 2009065778 A

**Littérature non-brevet citée dans la description**

- **SANCHEZ et al.** *Metabolic Engineering,* 2005, vol. 7, 229-239